# EUROPEAN PATENT APPLICATION

(11) **EP 4 810 109 A1**
(43) Date of publication of application: **23.09.2026**
(21) Application number: 26164412.4
(22) Date of filing: 12.03.2026
(51) Int. Cl.: A61N 5/10

(54) **METHOD AND SYSTEM FOR RADIOTHERAPY CONTROL**

(30) Priority: 18.03.2025 GB 202503950
(71) Applicant: Elekta Limited, Crawley, West Sussex RH10 9BL (GB)
(72) Inventor: Smith, Adrian, Crawley (GB); Lutz, Uli, Crawley (GB)
(74) Representative: Bollinghaus, Emer

(57) **Abstract**

A computer-implemented method is provided, for generating a first control signal for a radiotherapy system. The method comprises receiving an indicator of the occurrence of a trigger event and, in response to receiving said indicator of the occurrence of the trigger event, generating the first control signal. The first control signal is configured to control a transition of the radiotherapy system from a first power state to a second, different power state.

## Description

This disclosure relates to a control system and to a computer-implemented method of generating a control signal for a radiotherapy system, and in particular to a control system and a computer-implemented method that generate a control signal for a radiotherapy system, in response to a trigger event.

### Background

Radiotherapy can be described as the use of ionising radiation, such as X-rays, to treat a human or animal body. Radiotherapy is commonly used to treat cancer, for example to treat tumours within the body of a patient or subject. In such treatments, ionising radiation is used to irradiate, and thus destroy or damage, cells which form part of the tumour.

A radiotherapy system typically has many subsystems that may be powered or unpowered, and for some or each of which there may be more than one possible powered state. The respective states of the various subsystems give rise to the overall energy consumption of the radiotherapy system. When overall energy consumption for a radiotherapy system is high, this can lead to one or more of: increased financial cost, increased carbon footprint, and/or increased wear and tear on components of the radiotherapy system.

The present disclosure seeks to address these and other disadvantages encountered in the prior art.

### Summary

An invention is defined in the independent claims. Optional features are set out in the dependent claims.

### Figures

Specific embodiments are now described, by way of example only, with reference to the drawings, in which:
Figure 1 depicts a radiotherapy system according to the present disclosure;
Figure 2 depicts a radiotherapy system according to the present disclosure;
Figure 3 depicts a computer readable medium according to the present disclosure;
Figure 4 depicts a method according to the present disclosure;
Figure 5a depicts a first example power profile for a radiotherapy system, according to the present disclosure;
Figure 5b depicts a second example power profile for a radiotherapy system, according to the present disclosure;
Figure 5c depicts a third example power profile for a radiotherapy system, according to the present disclosure;
Figure 6 depicts possible inputs and outputs of a power consumption model according to a second aspect of the present disclosure;
Figure 7 depicts a method according to the present disclosure according to the second aspect of the present disclosure.

### Detailed Description

In overview, and without limitation, the application relates to controlling the transition of a radiotherapy system from a first power state to a second, different power state. For example, the first power state may be a relatively low power state and the second, different power state may be a relatively high power state, or vice versa.

The radiotherapy system may include any suitable number and combination of component parts. Its component parts may be comprised within a single physical entity or may be comprised within multiple physically separate entities. For example, the radiotherapy system may include a radiotherapy apparatus, which is operable for radiotherapy beam delivery to a patient. For example, the radiotherapy system may include an imaging device or imaging apparatus. For example, the radiotherapy system may include a KV generator. For example, the radiotherapy system may include an MV generator. For example, the radiotherapy system may include one or more of: magnets, focus solenoids, or magnetron magnets. For example, the radiotherapy system may include a gamma knife. For example, the radiotherapy system may include an MR subsystem.

The transition from the first power state to the second, different power state may be applicable to the entire radiotherapy system or to one or more component parts of the radiotherapy system. In some cases, generation and application of a control signal may cause different transitions in different respective parts of the radiotherapy system. By way of non-limiting example, a single control signal may cause one component part of the system to move from a relatively low power state to a relatively high power state and it may cause a respective other component part of the system to move from a relatively high power state to a relatively low power state, or to switch on or off.

In an example, the first power state is a relatively low power state and the second power state is a relatively high power state. The radiotherapy system may be configured for - or, may be configured to transition relatively quickly and/or directly to - radiotherapy beam delivery, when it is in the relatively high power state. Conversely, the radiotherapy may not be configured to transition as quickly and/or as directly from the relatively low power state, to radiotherapy beam delivery. Therefore, the methods and systems disclosed herein may provide a balance between reducing energy consumption, by placing the apparatus in the relatively low power state at an appropriate respective time or times, and providing efficient and timely radiotherapy beam delivery, by placing the apparatus in a ready state, corresponding to the relatively high power state, at an appropriate respective time or times.

The transition from a relatively low power state to a relatively high power state may be referred to as a "powering-up" step. The transition from a relatively low power state to a relatively high power state may be referred to as a "beam loading" step. When the radiotherapy system is delivering a radiotherapy beam, it will be in a beam-delivery power state, which may be higher than the relatively high power state referred to above. In other words, in the relatively high power state, the radiotherapy system may be configured so that the radiotherapy beam is loaded, and is ready to deliver, but is not currently being delivered.

A control signal for controlling the transition of the radiotherapy system, between a first power state and a second, different power state - for example, between a relatively low power state and a relatively high power state, or vice versa - may be generated in response to the occurrence of a trigger event. The trigger event may relate to the radiotherapy system. For example, it may comprise a external or internal event, relating to the radiotherapy system. A computer or other suitable controller or processor may be configured to receive an indicator of the occurrence of the trigger event, and to generate the control signal in response thereto. The control signal may be communicated to the radiotherapy system, to cause the transition from the first power state to the second, different power state.

A relatively low power state may comprise a state of the radiotherapy system, in which the machine is maintained at or above a predetermined temperature. The relatively low power state may comprise a "power save" state, wherein the apparatus is operational but not (presently) operating at a high enough power to immediately and/or directly load and deliver a beam of radiotherapy treatment, for example, in the relatively low power state, the magnets of the radiotherapy system may not be (sufficiently) energised - therefore, there may not be enough power - to immediately and/or directly load and deliver a beam of radiotherapy treatment. However, the predetermined temperature may ensure that it is possible for the apparatus to power-up, and to transition, relatively quickly, from that relatively low power state to the relatively high power state.

A relatively high power state may comprise a "hot state" or a "standby state", wherein the apparatus is maintained at a relatively high temperature and so would be ready to radiate (i.e., to deliver a radiotherapy beam) very quickly, for example, within seconds. The present inventors have recognised that a radiotherapy system may historically be kept in a hot, standby state for a relatively long time, within a working day. By reducing the time for which the apparatus is in a hot, standby state, overall energy consumption of the apparatus may be reduced. The methods and systems disclosed herein may enable this to be achieved in an efficient, and operationally streamlined manner.

A proposed workflow may be defined for the radiotherapy system. A proposed workflow may be patient-specific and/or appointment specific. A proposed workflow may comprise workflow data that comprises a schedule of upcoming treatment actions, such as "patient set-up", "patient imaging", "delivery of radiation according to treatment plan", etc. Each treatment action may be associated with one or more recommended operational states for the radiotherapy system. The workflow data may be described, therefore, as a schedule of upcoming operational states.

A control signal may be generated in accordance with a proposed workflow for the radiotherapy system. In other words, the control signal may be generated, in response to an indicator that a trigger event has occurred and/or by taking a proposed workflow for the radiotherapy system into account. The proposed workflow may include a radiotherapy beam delivery step. For example, a generated control signal may be configured - for example, may be generated, and/or timed, and/or communicated to the radiotherapy system - to cause the transition from a first, relatively low power state to a second, different, relatively high power state to occur just before - i.e., within a relatively short time frame before - the radiotherapy beam delivery is scheduled to occur, according to the proposed workflow. Since the transition from the relatively low power state to the relatively high power state may occur just before the radiotherapy beam delivery is scheduled to occur, a technical effect is achieved of reducing an amount of time for which the radiotherapy system is in a relatively high power state, and therefore consuming a relatively high amount of energy, but is not (yet) required to deliver a radiotherapy beam to a patient. Thus, according to the present disclosure, improvements may be achieved in relation to the operational efficiency of the radiotherapy system, and/or its environmental impact, and/or the wear and tear on at least some of its component parts.

The proposed workflow may include an end (or, completion) of the radiotherapy beam delivery. For example, a generated control signal may be configured - for example, may be generated, and/or timed, and/or communicated to the radiotherapy system - to cause the transition from a first, relatively high power state to a second, different, relatively low power state to occur just after - i.e., within a relatively short time frame after - the radiotherapy beam delivery is completed, according to the proposed workflow. Since the transition from the relatively high power state to the relatively low power state may occur just after the radiotherapy beam delivery has occurred, a technical effect is achieved of reducing an amount of time for which the radiotherapy system is in a relatively high power state, and therefore consuming a relatively high amount of energy, but is no longer required to deliver a radiotherapy beam to a patient. Thus, improvements may be achieved, according to the present disclosure, in relation to the operational efficiency of the radiotherapy system, and/or its environmental impact, and/or the wear and tear on at least some of its component parts.

It has been recognised by the present inventors that a trigger event may be used to initiate and/or to guide a computer-implemented method for generating control signals, for readying a radiotherapy system for radiotherapy beam delivery to a patient. Thus, a streamlined, repeatable, and efficient approach to control of a radiotherapy system is possible.

It has been recognised by the present inventors that workflows for radiotherapy system are typically more reliable and/or more detailed than was historically the case. For example, it has been recognised that workflows for radiotherapy devices typically comprise one or more defined operational steps, including but not limited to a (finite) beam delivery step. One or more of those defined operational steps may have a respective expected or demanded time frame associated with it/them. The methods and systems disclosed herein may make use of such defined operational steps, to obtain a more accurate picture of when radiotherapy beam delivery is actually to be expected, and/or when it will be completed, when a patient is present at a patient treatment location. For example, the methods and systems disclosed herein need not rely solely on a scheduled patient appointment time, in order to schedule powering-up of the radiotherapy system for beam delivery or to schedule powering-down of the radiotherapy system after beam delivery. The methods and systems disclosed herein may therefore avoid powering-up the radiotherapy device too early, for example when it knows that additional steps - such as, but not limited to: movement of the operator of the radiotherapy system, and/or patient imaging, which may be adaptive imaging, and/or patient position correction (PPC), and/or image review, and/or workflow review, and/or workflow amendment, which may be adaptive amendment - are planned, or at least are likely, before the radiotherapy beam delivery will be required, for a specific patient. The methods and systems disclosed herein may avoid keeping the radiotherapy system powered-up for too long, after it has delivery its radiotherapy beams and it no longer needed for beam delivery to a specific patient.

These and other advantages will be discussed in the present disclosure.

Disclosed herein is a computer-implemented method of generating a first control signal for a radiotherapy system, the method comprising receiving an indicator of the occurrence of a trigger event and, in response to receiving said indicator of the occurrence of the trigger event, generating the first control signal, wherein the first control signal is configured to control a transition of the radiotherapy system from a first power state to a second, different power state.

The radiotherapy system may include a radiotherapy beam delivery apparatus, or radiotherapy beam delivery device. The radiotherapy system may include an imaging apparatus, or imaging device.

The first power state may be a relatively low power state and the second, different power state may be a relatively high power state, or vice versa. The relatively high power state may be a preparatory state, for radiotherapy beam delivery. For example, the transition of the radiotherapy system from the first power state to the second, different power state may correspond to a beam loading step, or a powering-up step, for the radiotherapy system, in advance of radiotherapy beam delivery. For example, the transition of the radiotherapy system from the first power state to the second, different power state may correspond to a beam-cancelling or beam termination step , or a powering-down step, for the radiotherapy system, for example after delivery of radiotherapy beams is complete, for a particular patient .

Because the control signal may be generated in response to receiving an indicator of the occurrence of the trigger event, an automated (or, at least semi-automated) method for controlling a change of power state of the radiotherapy system, for example in readiness for radiotherapy treatment beam delivery, or to power down after radiotherapy treatment beam delivery, is achievable.

Because an occurrence of a trigger event is used to prompt the generation of the control signal, the generation of that control signal may be based on what is actually happening (or, on what has actually happened), for the radiotherapy system and/or for the patient who is to be treated and/or for the operator of the radiotherapy system. Therefore, better temporal alignment may be achieved between the operational state of the radiotherapy system and the progress of a patient's radiotherapy appointment, or treatment session. This enables the radiotherapy system to be in a relatively high power state for a shorter amount of time. Thus, the operation of the radiotherapy system may be more efficient and more environmentally sustainable, through reduced energy consumption.

For example, the trigger event may comprise a physical event. For example, the trigger event may comprise an event that occurs external to the radiotherapy system such as, but not limited to, the closing of a door or other barrier, securing a patient treatment location from an adjacent area. For example, the trigger event may comprise - or, may be indicative of - an operator of the radiotherapy and/or another person or entity (who is not the patient to be treated), vacating a patient treatment location. The trigger event may comprise an event that occurs on or within the radiotherapy system. For example, the trigger event may comprise a beam loading step. For example, it may comprise a predetermined movement or positioning of a part of the radiotherapy system. The trigger event may correspond to step within, or to a completion or part-completion of, a workflow that has been proposed or defined for the radiotherapy system, for the treatment of a particular patient.

Although "a first" power state and "a second" power state are referred to above, the method may comprise generating one or more control signals, configured to transition the radiotherapy system between more than two different respective power states. For example, there may be multiple discrete or continuous power levels, corresponding to one or more lower power state(s) of the part or all of the radiotherapy system. For example, there may be multiple discrete or continuous power levels, corresponding to one or more higher power state(s) of at least part of the radiotherapy system.

A "power state" may comprise defined respective power levels, or power states, for two or more component parts of the radiotherapy system. For example, it may be possible for the change in power state for the radiotherapy system to comprise an increase in power level/state for one component part and a decrease in power level/state for another component part. For example, it may be possible for the change in power state for the radiotherapy system to comprise a change in power level/state of a first amount for a first component of the radiotherapy system and a change in power level/state of a second, different amount for a second component of the radiotherapy system.

A relatively high, or "yet-higher", power state may be defined for beam delivery. There may be more than one beam delivery power state.

Optionally, the first control signal is configured to control a timing of the transition of the radiotherapy system, from the first power state to the second, different power state. For example, the control signal may be configured to provide a pre-determined delay, or time lapse, between the occurrence of the trigger event and the transition of the radiotherapy system to the second, different power state. For example, the control signal may be configured to initiate the transition of the radiotherapy system to the second, different power state when one or more subsequent events or steps, after the trigger event, are detected.

Optionally, a proposed workflow may be defined for the radiotherapy system. The method may comprise obtaining an indicator of the proposed workflow, and configuring the first control signal in accordance with said proposed workflow.

A controller may be provided that has access to a proposed workflow for the radiotherapy system, and which can issue one or more control signals for changing a power state of the radiotherapy system. For example, such a controller may comprise an integrated console. The controller may give the user (i.e., an operator of the radiotherapy system) access to a range of information about the patient and/or about the treatment plan for the current treatment sessions and/or other treatment sessions. The controller may provide access to images, for example two-dimensional and/or three-dimensional images, relating to the patient. The controller may enable the user to prompt or change or configure beam delivery, based at least in part on the proposed workflow.

The proposed workflow may enable a determination to be made, regarding the content of, and/or the timing of the generation of, and/or the timing of the communication of, the control signal. The proposed workflow may inform (i.e., it may provide information that contributes to) one or more aspects of the control signal. For example, it may inform the power level of the second power state, to which part or all of the radiotherapy system will be transitioned. For example, it may inform the identity and/or the location and/or the orientation of the radiotherapy beam or beams that should be delivered to the patient. For example, it may inform the duration for which the radiotherapy beam or beams should be delivered to the patient. For example, it may inform the powering-down of the system, after beam delivery. For example, it may inform imaging, to be done by a part or parts of the radiotherapy system.

Optionally, the proposed workflow comprises a radiotherapy beam delivery step for the radiotherapy system.

The first control signal may be configured to cause said transition of the radiotherapy system to occur, from the first power state to the second power state, before a time at which the radiotherapy beam delivery step should occur, in accordance with the proposed workflow. Therefore, for example, the method may enable a transition to occur, from a lower power state to a higher power state, on time for the radiotherapy beam to be delivered, without introducing a delay into a workflow or treatment plan for a patient. This may be balanced, however, by ensuring that the transition does not occur too early, thereby avoiding or at least reducing overall energy consumption, by the radiotherapy system.

The first control signal may be configured to cause the transition of the radiotherapy system to occur, from the first power state to the second power state, after a time by which the radiotherapy beam delivery step should be complete, in accordance with the proposed workflow. Therefore, for example, the method may enable a transition to occur, from a higher power state to a lower power state, soon after the radiotherapy beam has been delivered, thereby avoiding or at least reducing overall energy consumption, by the radiotherapy system.

Optionally, the time at which the radiotherapy beam delivery step should occur, and/or the time by which the radiotherapy beam delivery step should be complete, is defined based on one or more of: an indicator of the occurrence of the trigger event; an indicator that the proposed workflow has begun; an indicator that a preceding step in the proposed workflow has begun; an indicator that a preceding step in the proposed workflow is complete; or, an indicator that an input has been received, from an operator of the radiotherapy system. Therefore, the method may use actual events, and/or expected events, within a workflow, treatment plan, and/or treatment appointment, to achieve better control of the powering-up and/or powering-down of the radiotherapy system. The time at which beam delivery should occur and/or the time by which it should be complete may each be defined as an instantaneous time, or as a time window or time period, or as a time deadline.

Optionally, the first control signal may be configured to reduce (as compared to conventional approaches) a time period (T_{P}) that occurs between a time (T_{T}) at which the transition of the radiotherapy system occurs, from the first power state to the second power state, and the time at which the radiotherapy beam delivery step should occur, in accordance with the proposed workflow. Thus, for example, the method may reduce the time for which the radiotherapy system is consuming a relatively high amount of energy per unit time, but is not being operated for radiotherapy beam delivery.

Optionally, the time period (T_{P}) may be configured to provide at least a predetermined temporal buffer, between the time (T_{T}) at which the transition of the radiotherapy system occurs, from the first power state to the second power state, and the time at which the radiotherapy beam delivery step should occur, in accordance with the proposed workflow. Thus, some redundancy may be built into the disclosed method, to ensure the radiotherapy treatment beam will reliably be loaded and ready, on time for a scheduled radiotherapy beam delivery (or, on time for another operational event that requires the second, different power state.)

Optionally, the proposed workflow comprises an interim step, preceding the radiotherapy beam delivery step. The interim step may be scheduled to occur during a time interval (T_{INT}), in accordance with the proposed workflow. The control signal may be configured so that time period (T_{P}) is comprised within, or occurs just after, or at least partially overlaps in time with, said time interval (T_{INT}). Thus, the transition of the radiotherapy system, to the second power state, may occur in parallel with one or more other steps that are planned, for the radiotherapy system, before beam delivery. Therefore, although the method disclosed herein may reduce the size of the time period (T_{P}) for which the radiotherapy system is in the second power state but not being used for beam delivery, as compared to prior art approaches, it nonetheless may ensure that the transition to the second power state is achieved in a timely manner, and so substantially does not introduce delay or lag into the treatment delivery or workflow for the patient.

Optionally, a proposed workflow is defined for the radiotherapy system, wherein the proposed workflow includes an operator exit step, which requires an operator of the radiotherapy system to exit a patient treatment location. The operator may be a human operator. The operator exit step may be a trigger event. The operator exit step may include the closing of a door or other barrier, between the patient treatment location and another area, such as a control area or apparatus-operating location.

Optionally, a proposed workflow is defined for the radiotherapy system. The radiotherapy beam delivery step may be scheduled, within the proposed workflow, to occur after the trigger event. The first control signal may be configured to control the transition of the radiotherapy system to occur, from the first power state to the second, different power state, after the trigger event and before the radiotherapy beam delivery step. The first control signal may be configured not to initiate the transition immediately after the trigger event. There may be, for example, a time delay between the trigger event and the transition. The control signal may be configured such that - and/or the method disclosed herein may otherwise require that - an additional step should occur, and/or an indicator or a signal should be received, before the transition is initiated, after the trigger event.

Optionally, a step defined in a proposed workflow for the radiotherapy system may correspond to the trigger event, the occurrence of which may prompt the generation of the first control signal. In other words, the trigger event may be the same as, or may be connected to, a step defined in the proposed workflow. For example, the proposed workflow may include an operator exit step, which requires an operator of the radiotherapy system to exit a patient treatment location. The trigger event in such an example may also be (or, may be connected to) the operator exit step. For example, an indicator of the occurrence of the trigger event in such an example may be a detection that a door or other barrier has closed, upon exit of the operator from the patient treatment location. For example, an indicator of the occurrence of the trigger event in such an example may be a detection of a movement of the operator, from the patient treatment location to an apparatus-operating location, and/or the detection of the presence of the operator, at a location remote from the patient treatment location.

Optionally, the proposed workflow comprises a set-up step, which may demand a physical presence of an operator of the radiotherapy system, at a patient treatment location. This may occur before the trigger event.

Optionally, the trigger event may comprise more than one event, in combination. Optionally, the trigger event may correspond to one or more detectable or measurable parameters meeting, exceeding, or falling below one or more respective thresholds.

Optionally, a proposed workflow is defined for the radiotherapy system, wherein the proposed workflow includes an interim step, which is scheduled to occur after the trigger event and before a radiotherapy beam delivery step; wherein the first control signal is configured to control the transition of the radiotherapy system to occur, from the first power state to the second, different power state, during or after the interim step of the proposed workflow.

Optionally, the interim step comprises at least one of: patient imaging, which may be adaptive imaging; image review; patient position correction; review of a proposed beam delivery plan; and/or adaptation of a proposed beam delivery plan. The interim step may have a time frame, or duration, associated with it, within the proposed workflow. An input from an operator, or another input, may be provided to the controller that is implementing the method disclosed herein, to indicate that the interim step has begun, and/or that the interim step is complete. The controller itself may detect that the interim step has begun, and/or that the interim step is complete.

Optionally, the method disclosed herein comprises receiving an indicator of a change to a proposed workflow for the radiotherapy system and generating the first control signal in accordance with said indicator of a change to the proposed workflow. For example, an operator and/or a controller may suggest or demand a change to the proposed workflow, in response to an interim step such as, but not limited to, patient imaging, image review, patient position correction, review of a proposed beam delivery plan, input from the patient, and/or adaptation of a proposed beam delivery plan. The method disclosed herein may provide opportunity for input of a suggested or demanded change to a proposed workflow for the radiotherapy system, before the first control signal is generated. Thus, the method disclosed herein may be adaptive.

Optionally, the method disclosed herein comprises receiving an indicator of a change to a proposed workflow for the radiotherapy system and amending a previously-generated first control signal, in accordance with said indicator of a change to the proposed workflow. For example, an operator and/or a controller may suggest or demand a change to the proposed workflow, in response to an interim step such as, but not limited to, patient imaging, which may be adaptive imaging, image review, patient position correction, review of a proposed beam delivery plan, input from the patient, and/or adaptation of a proposed beam delivery plan. The operator may use any suitable input system for suggesting or demanding a change to the proposed workflow. For example, they may do so via a display or console, such as an integrated console. The method disclosed herein may generate the first control signal and may provide opportunity, after said generation, for input of a suggested or demanded change to a proposed workflow for the radiotherapy system, and amendment of the previously-generated first control signal, before that amended first control signal is communicated to the radiotherapy system. Thus, the method disclosed herein may be adaptive.

Optionally, the method disclosed herein comprises generating a second control signal, wherein the second control signal is configured to control a transition of the radiotherapy system from the second power state to the first power state. For example, the second control signal may be generated in response to the receipt of an indicator of the occurrence of a trigger event. That trigger event may be a second trigger event, which may be different to the (first) trigger event that prompted the generation of the first control signal. For example, the second control signal may be generated in response to an indicator that some or all steps of a proposed workflow for the radiotherapy system have been carried out. For example, it may be generated in response to an indicator that all radiotherapy beam delivery within the proposed workflow has been completed. For example, it may be generated in response to an indicator that there should be a pause in the delivery of radiotherapy beams to the patient, in accordance with the proposed workflow and/or in accordance with another input. Thus, the method disclosed herein may enable a timely powering-down step or steps, to help reduce overall energy consumption for the radiotherapy system.

Optionally, the method comprises receiving an input from an operator of the radiotherapy system, and generating a third control signal, wherein the third control signal is configured to control a transition of the radiotherapy system from the first power state to the second, different power state, or vice versa, or between any two power states for the radiotherapy system, in accordance with said input. For example, the method may enable a user-override of a proposed workflow or of a proposed operation or control of the radiotherapy system. For example, the third control signal may be combined with either the first control signal and/or the second control signal, to provide an amended control signal for the radiotherapy system, at an appropriate time or times.

Optionally, the method comprises outputting a human-readable indicator of at least one of: a present power state of the radiotherapy system; and/or, a scheduled power state of the radiotherapy system. The human-readable indicator may be configured to prompt the operator (or, another person or entity) to consider whether an input from them is appropriate. For example, it may prompt an operator to initiate a power-up or power-down step, if the present or scheduled power state is deemed inappropriate. For example, it may prompt the operator to provide an input if they become aware of a change to the workflow or operational schedule for the radiotherapy system, of which the controller is not yet aware.

Optionally, the method comprises communicating the first control signal, to implement said transition of the radiotherapy system from the first power state to the second, different power state. In other words, the method may comprise causing the transition to occur, in accordance with the generated first control signal.

Optionally, the method comprises causing a radiotherapy beam to be delivered to a patient. This may cause the radiotherapy system to further transition, from the second power state to a beam-delivery power state. The method may comprise causing multiple radiotherapy beams to be delivered, in succession and/or simultaneously.

Disclosed herein is a control system for a radiotherapy system, the control system being configured to receive an indicator of the occurrence of a trigger event and, in response to receiving said indicator of the occurrence of the trigger event, generate a first control signal, wherein the first control signal is configured to control a transition of the radiotherapy system from a first power state to a second, different power state. The first power state may be higher than the second power state, or vice versa. The higher power state may be a preparatory state, for radiotherapy beam delivery.

Optionally, the control system is configured to communicate the first control signal, to implement said transition of the radiotherapy system from the first power state to the second, different power state. In other words, the control system may be configured to cause the transition, in accordance with the generated first control signal.

The control system may comprise a computer or processor or microprocessor or any other suitable general-purpose controller or specific-purpose controller. Although described as a 'system', the control system may comprise a single entity or may comprise any number and arrangement of suitable component parts. The control system may comprise a console, such as an integrated console. The control system may comprise a display and/or a user input interface.

Optionally, the control system is configured to cause a radiotherapy beam to be delivered to a patient, for example when the radiotherapy system is in a higher power state. In other words, the control system may be configured to control delivery of a radiotherapy beam or beams to a patient, in accordance with the generated first control signal.

The control system may be configured to carry out a method comprising any of the optional and/or non-optional steps detailed above.

Disclosed herein is a method of operating a radiotherapy system, the method comprising receiving an indicator of the occurrence of a trigger event, relating to the radiotherapy system, generating a first control signal, in response to receiving said indicator of the occurrence of the trigger event, and communicating the first control signal, to implement a transition of the radiotherapy system from a first power state to a second, different power state.

Optionally, the method comprises controlling the radiotherapy system to transition from the second, power state to a third power state, wherein the radiotherapy system delivers a radiotherapy beam or beams when it is in the third power state. The third power state may comprise a beam-delivery power state.

Disclosed herein is a method of delivering a radiotherapy beam to a patient, the method comprising receiving an indicator of the occurrence of a trigger event, relating to the radiotherapy system, communicating a first control signal, generated in response to the indicator of the occurrence of the trigger event, to implement a transition of the radiotherapy system from a first power state to a second power state, and causing a radiotherapy beam to be delivered to a patient.

The step of causing the radiotherapy beam to be delivered to a patient may comprise causing the radiotherapy system to transition from the second power state to a third, beam-delivery power state.

Optionally, the method of delivery comprises delivering multiple radiotherapy beams to the patient. Optionally, the method of delivery comprises delivering a radiotherapy beam or beams to each of multiple locations, on and/or in the patient's body. The method may comprise controlling the type and/or the number and/or the sequence of radiotherapy beams that is delivered to the patient's body.

Disclosed herein is a computer-implemented method of generating a control signal for a radiotherapy system, to control a transition of the radiotherapy system from a first power state to a second, different power state, wherein said transition is scheduled to occur after the occurrence of a trigger event, and to precede a radiotherapy beam delivery step, by the radiotherapy system.

Optionally, the method comprises generating the control signal to control a timing of the transition of the radiotherapy system from the first power state to the second power state. For example, the second power state may be a relatively high power state and the control signal may determine that the radiotherapy system is in the second power state for a relatively small amount of time, before the radiotherapy beam delivery step. For example, it may determine that the radiotherapy system is in the second, higher power state for no more than a predetermined time period (Tₚ), before the radiotherapy beam delivery step.

According to a second aspect of the present disclosure, also disclosed herein is a computer-implemented method for generating a power consumption metric for a radiotherapy device comprising a plurality of components. The method comprises obtaining machine state data, the machine state data being indicative of which operational state of a plurality of possible operational states the radiotherapy device is currently operating in; retrieving machine configuration data comprising component data and calibration data, the component data identifying at least a subset of the plurality of components and the calibration data comprising at least one power usage value for each of the at least a subset of the plurality of components; and generating, based on the machine state data and the machine configuration data, a power consumption metric for the radiotherapy device.

Optionally, each operational state of the plurality of possible operational states is associated with a respective subset of the plurality of components; and retrieving the machine configuration data comprises retrieving, based on the obtained machine state data: component data identifying a subset of components associated with the operational state the machine is currently operating in; and calibration data comprising at least one power usage value for each of the subset of the plurality of components.

Optionally, particular components of the plurality of components are associated with a particular operational state if they consume power while the radiotherapy device is operating in the particular operational state.

Optionally, the at least one power usage values are indicative of the amount of power required to operate the associated component.

Optionally, the power consumption metric is indicative of the amount of power currently being consumed by the radiotherapy device.

Optionally, the method may further comprise displaying, via a user interface, the power consumption metric to a user.

Optionally, the method may further comprise retrieving workflow data, the workflow data comprising a schedule of upcoming treatment actions, each treatment action being associated with one or more recommended operational states; wherein the method further comprises generating, based on the workflow data, a suggestion for a user to modify the current operational state.

Optionally, the suggestion is that the user modify the current operational state to a recommended operational state associated with a currently scheduled, or next scheduled, treatment action.

Optionally, the next scheduled treatment action is a break in treatment associated with a "low-power mode" machine state; and the suggestion is that the user modify the current operational state to the "low-power mode" machine state.

Optionally, the method further comprises displaying to the user, via a user interface: the suggestion; and a selectable control item which, when selected by the user, causes the radiotherapy device to enter the recommended operational state.

Optionally, the calibration data comprises, for a first component of the plurality of components, both a first and a second power usage value, wherein the first power usage value is associated with a first operational state and the second power usage value is associated with a second operational state.

Optionally, the first component uses power in accordance with the first power usage value while the machine is in the first operational state, and the second component uses power in accordance with the second power usage value while the machine is in the second operational state.

Disclosed herein is a radiotherapy device, one or more processors, and a computer-readable medium comprising instructions which, when performed by the one or more processors, cause the one or more processors to perform the method according to any one or more of methods disclosed herein.

Optionally, the system may comprise a user interface configured to display a power consumption metric to a user.

Disclosed herein is a transitory or non-transitory computer readable medium storing instructions which, when executed by one or more processors of a computing device, cause the computing device to carry out the method according to any one or more of methods disclosed herein. The instructions may comprise any suitable combination of optional and/or non-optional steps, as disclosed herein.

The methods and systems disclosed herein may be understood further in reference to the drawings.

### Radiotherapy device

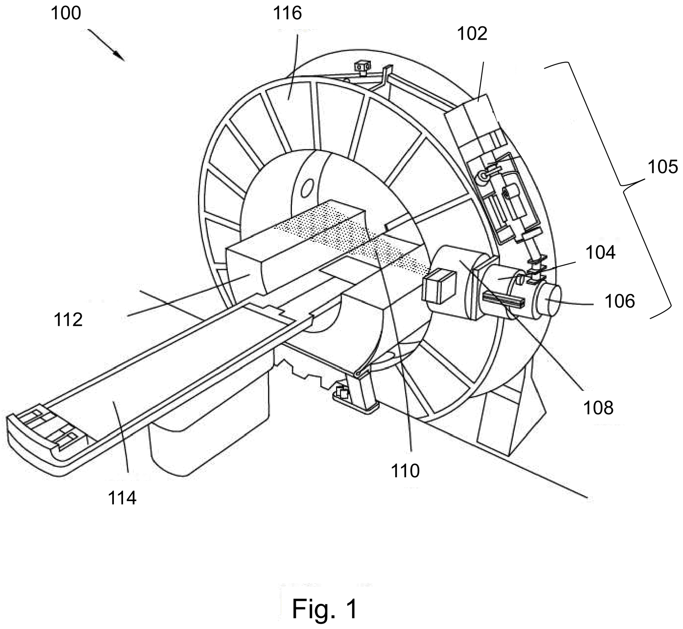

Figure 1 depicts a radiotherapy device 100, by way of example only. The present disclosure may be applied to other types of radiotherapy system.

The radiotherapy device 100 is suitable for delivering, and configured to deliver, a beam of radiation 110 to a patient during radiotherapy treatment. The device 110 and its constituent components will be described generally for the purpose of providing useful accompanying information for the present application. The device 100 depicted in figure 1 is in accordance with the present disclosure and is suitable for use with the disclosed systems and apparatuses. While the device 100 in figure 1 is an MR-linac, in implementations of the present disclosure the device 100 may be another type of radiotherapy device, for example a linac device with a different imaging capability.

In overview, the radiotherapy device 100 comprises a source of radiation 105 and an imaging apparatus 112. The source of radiation 105 is coupled to a rotatable gantry 116. The device 100 comprises a patient positioning apparatus which comprises a patient positioning surface 114 on which a patient may be positioned. Before treatment, the patient is positioned on the surface 114, and the patient positioning apparatus may be used to position the patient in an appropriate position for the treatment, for example according to a reference image on which the patient's treatment plan is based. During treatment, the source of radiation 105 delivers radiation to the patient according to the patient's treatment plan.

The source of radiation 105 is configured to generate a beam of radiation 110, and in particular a beam of therapeutic radiation. The radiation source 105 is attached to the rotatable gantry 16 so as to rotate with the gantry 16. In this way, the radiation source is rotatable around the patient so that the beam 110 can be applied from different angles around the gantry 116. The source of radiation 105 may comprise a beam generation system comprising a linear accelerator (linac). For such a linac device, the beam generation system may comprise a source of RF energy 102, an electron gun 106, and a waveguide 104.

The source 102 of radiofrequency waves, such as a magnetron, is configured to produce radiofrequency waves. The source 102 of radiofrequency waves is coupled to the waveguide 104, for example via a circulator, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

Once the electrons have been accelerated, they travel toward a heavy metal target which, when impacted by the electrons, generates a beam of high energy photons, forming a radiation beam 110. When the electrons strike the target, X-rays are produced in a variety of directions. The device 100 comprises collimation apparatus 108. The collimation apparatus 108 may comprise a primary collimator. The primary collimator is configured to block X-rays travelling in certain directions and pass only forward travelling X-rays to produce a treatment beam 110. The X-rays may be filtered and may pass through one or more ion chambers for dose measuring. The collimation apparatus 108 may additionally comprise beam shaping apparatus such as a multi-leaf collimator (MLC). The beam can be shaped in various ways by the beam-shaping apparatus. The source of radiation is configured to direct the beam 110 of therapeutic radiation, having been appropriately filtered and shaped by the collimation apparatus 108, toward a patient positioned on the patient support surface 114.

The device 100 comprises an imaging apparatus 112 or 'image acquisition apparatus'. The depicted imaging apparatus 112 is an MR imaging apparatus, though the imaging apparatus may take other forms, for example a cone beam computed tomography (CBCT) apparatus. The MR imaging apparatus 112 is shown in cross-section in the diagram. The imaging apparatus 112 is configured to generate imaging data. The imaging data may comprise images of the patient. The imaging apparatus 112 is therefore configured to obtain images of a patient positioned on the patient support surface 114. The imaging data generated by the imaging apparatus 112 may be used to generate a reference image to enable treatment planning, and/or may be used during the delivery of therapeutic radiation to help guide the beam of radiation 110 or to provide an input into motion management and real-time adaptive radiotherapy techniques.

Figure 1 and its accompanying description herein is provided to give context to the application and to facilitate understanding of the present invention. In addition to the components described in overview above, a radiotherapy device also comprises many other functions, components and sub-systems as will be understood by the skilled person.

### Radiotherapy (Computing) System

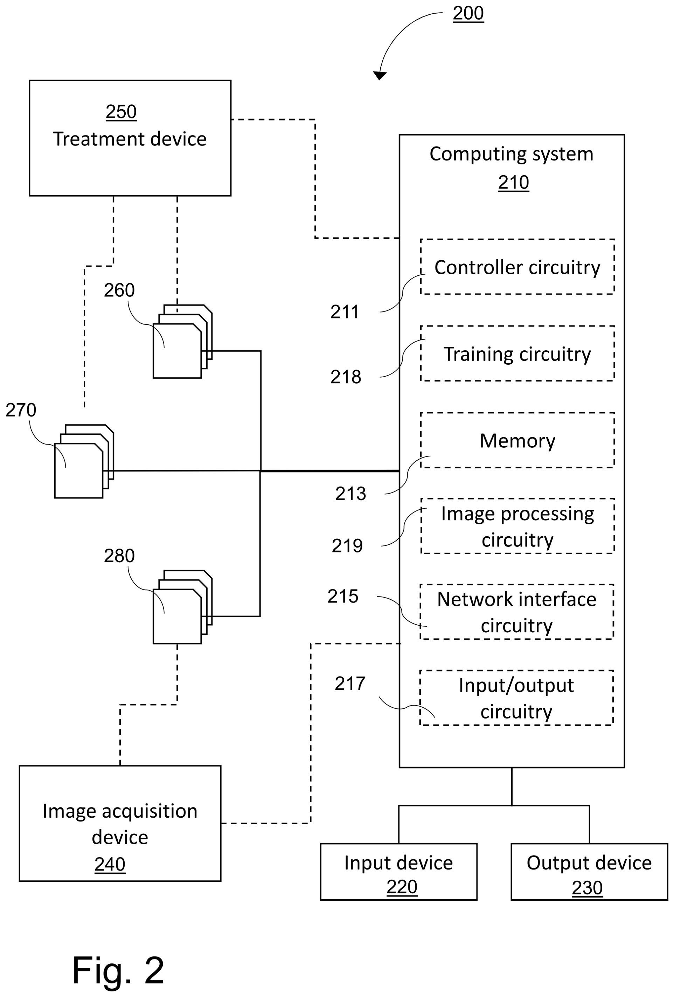

Figure 2 illustrates a block diagram of one implementation of a radiotherapy system 200. The radiotherapy system 200 comprises a computing system 210 within which a set of instructions, for causing the computing system 210 to perform any one or more of the methods discussed herein, may be executed.

The computing system 210 shall be taken to include any number or collection of machines, e.g. computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STBEAM), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 210 includes controller circuitry 211 and a memory 213 (e.g., read-only memory (ROM), flash memory, dynamic random access memory (DRAM) such as synchronous DRAM (SDRAM) or Rambus DRAM (RDRAM), etc.). The memory 213 may comprise a static memory (e.g., flash memory, static random access memory (SRAM), etc.), and/or a secondary memory (e.g., a data storage device), which communicate with each other via a bus (not shown).

Controller circuitry 211 represents one or more general-purpose processors such as a microprocessor, central processing unit, accelerated processing units, or the like. More particularly, the controller circuitry 211 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 211 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 211 is configured to execute the processing logic for performing the operations and steps discussed herein.

The computing system 210 may further include a network interface circuitry 215. The computing system 210 may be communicatively coupled to an input device 220 and/or an output device 230, via input/output circuitry 217. In some implementations, the input device 220 and/or the output device 230 may be elements of the computing system 210. The input device 220 may include an alphanumeric input device (e.g., a keyboard or touchscreen), a cursor control device (e.g., a mouse or touchscreen), an audio device such as a microphone, and/or a haptic input device. The output device 230 may include an audio device such as a speaker, a video display unit (e.g., a liquid crystal display (LCD) or a cathode ray tube (CRT)), and/or a haptic output device. In some implementations, the input device 220 and the output device 230 may be provided as a single device, or as separate devices.

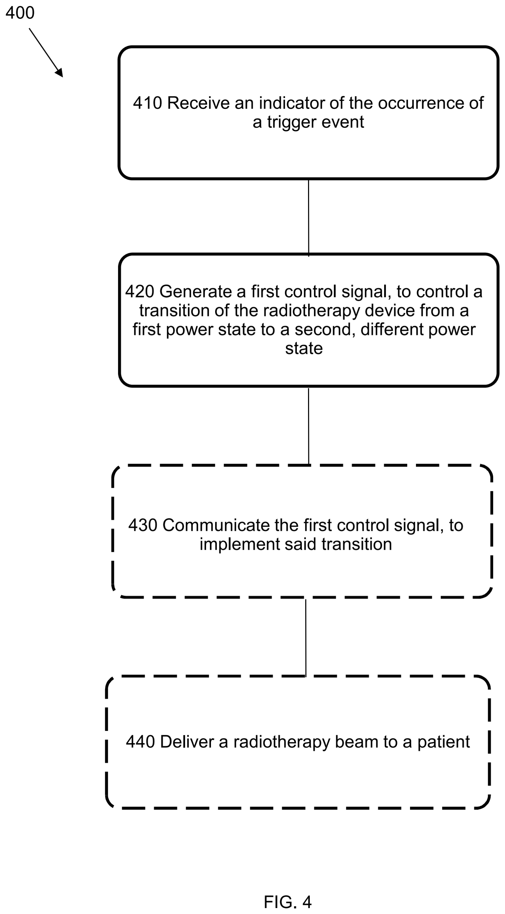

In some implementations, computing system 210 includes training circuitry 218. The training circuitry 218 is configured to train a method of generating a control signal for a radiotherapy system. For example, training circuitry 218 may train a model for performing a method of generating a control signal for a radiotherapy system, the control signal being configured to control a transition of the radiotherapy system from a first, relatively low power state, to a second, relatively high power state, or vice versa. The second, relatively high power state may be a preparatory state, in which the radiotherapy system is ready for beam delivery (but is not in a beam-delivery state, which may correspond to a third, yet-higher power state). The model may comprise a deep neural network (DNN), such as a convolutional neural network (CNN) and/or recurrent neural network (RNN). Training circuitry 218 may be configured to execute instructions to train a model that can be used to generate a control signal for a radiotherapy system, the control signal being configured to control a transition of the radiotherapy system from a first, power state, to a second, different power state, as described with reference to Figure 4, below. Training circuitry 218 may be configured to access training data and/or testing data from memory 213 or from a remote data source, for example via network interface circuitry 215. In some examples, training data and/or testing data may be obtained from an external component, such as image acquisition device 240 and/or treatment device 250. In some implementations, training circuitry 218 may be used to update, verify and/or maintain the model for generating a control signal for a radiotherapy system, the first control signal being configured to control a transition of the radiotherapy system from a first power sate, to a second, different power state.

In some implementations, the computing system 210 may comprise image processing circuitry 219. Image processing circuitry 219 may be configured to process image data 280 (e.g. images, or imaging data), such as medical images obtained from one or more imaging data sources, a treatment device 250 and/or an image acquisition device 240. Image processing circuitry 219 may be configured to process, or pre-process, image data. For example, image processing circuitry 219 may convert received image data into a particular format, size, resolution or the like. In some implementations, image processing circuitry 219 may be combined with controller circuitry 211.

In some implementations, the radiotherapy system 200 may further comprise an image acquisition device 240 and/or a treatment device 250. The image acquisition device 240 and the treatment device 250 may be provided as a single device. In certain implementations, image acquisition device 240 and treatment device 250 are a combined radiotherapy device capable of delivering both therapeutic radiation and acquiring imaging data, such as the device disclosed herein in the examples of Figure 1. In some implementations, treatment device 250 is configured to perform imaging, for example in addition to providing treatment and/or during treatment. The treatment device 250 comprises the main radiation delivery components of the radiotherapy system, such as, for example, a linear accelerator (linac).

Image acquisition device 240 may be configured to perform positron emission tomography (PET), computed tomography (CT), magnetic resonance imaging (MRI), and/or any other suitable imaging technique(s). Image acquisition device 240 may be configured to output image data 280, which may be accessed by computing system 210. Treatment device 250 may be configured to output treatment data 260, which may be accessed by computing system 210.

Computing system 210 may be configured to access or obtain treatment data 260, planning data 270 and/or image data 280. Treatment data 260 may be obtained from an internal data source (e.g. from memory 213) or from an external data source, such as treatment device 250 or an external database. Planning data 270 may be obtained from memory 213 and/or from an external source, such as a planning database. Planning data 270 may comprise information obtained from one or more of the image acquisition device 240 and the treatment device 250.

The various methods described above may be implemented by a computer program. The computer program may include computer code (e.g. instructions) 310 arranged to instruct a computer to perform the functions of one or more of the various methods described above. The steps of the methods described above may be performed in any suitable order. For example, the step of generating a first control signal, configured to control a transition from the first power state, to the second, different power state, may be performed before, after, simultaneously, or substantially simultaneously with a step of generating or communicating a signal to implement an interim step, scheduled to happen before a radiation beam delivery step. For example, the step of communicating the generated control signal to a radiotherapy device may occur after, simultaneously, or substantially simultaneously with a step of generating the control signal. For example, the step of amending a control signal, in accordance with a change to a proposed workflow for a radiotherapy device, may occur before, after, simultaneously, or substantially simultaneously with a step of outputting a human-readable readable indicator of at least one of: a present power state of the radiotherapy system; and, a scheduled power state of the radiotherapy system.

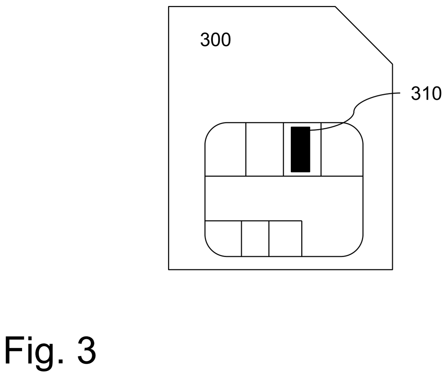

The computer program and/or the code 310 for performing such methods may be provided to an apparatus, such as a computer, on one or more computer readable media or, more generally, a computer program product 300)), depicted in Figure 3. The computer readable media may be transitory or non-transitory. The one or more computer readable media 300 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions 310 may also reside, completely or at least partially, within the memory 213 and/or within the controller circuitry 211 during execution thereof by the computing system 210, the memory 213 and the controller circuitry 211 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

### Method of Generating a Control Signal

Figure 4 is a flowchart depicting a method 400 according to the present disclosure. The method 400 depicted is a computer implemented method. In this example, the computing system 210 is configured to implement the method 400 of Figure 4, to generate a control signal for the radiotherapy device 100 shown in Figure 1. But any suitable controller, processor, or microprocessor, such as but not limited to those described hereabove, may be configured to implement the method 400 of Figure 4. And the method 400 of Figure 4 may be used to generate control signals for any suitable radiotherapy device or radiotherapy system.

At block 410, the computing system 210 is configured to receive an indicator of the occurrence of a trigger event. In this example, the trigger event is the closing of a door, to secure the patient treatment location, in which the radiotherapy device 100 and patient are located, from the surrounding areas. This closing of the door is required, for example, to help prevent radiation travel out of the patient treatment location, in order to protect the device operator and others. In this example, a sensor is configured to detect the door closing, and to communicate a signal to the computing system 210 that the trigger event (the door closing) has occurred.

In other examples, other trigger events may be used. For example, the trigger event may relate to the movement of the device operator, away from the patient treatment location and/or towards a device operating area. For example, the trigger event may relate to the positioning of the patient and/or to the cessation (stopping) of movement of a patient treatment surface, indicating that the patient is now in a correct position for treatment. For example, the trigger event may comprise a beam loading step. For example, the trigger event may correspond to a workflow beginning, or to a specific point in a workflow being reached or completed, for the radiotherapy device 100.

At block 420, the computing system 210 is configured to generate a first control signal, upon receipt of the indicator that the trigger event has occurred. The first control signal is configured to control a transition of the radiotherapy device 100 from a first power state to a second, different power state. In this example, the second power state is a higher power state, relative to the first power state. In this example, the transition is to occur before the delivery of radiotherapy beams to a patient, as part of radiotherapy treatment.

In other examples, the second power state may be a lower power state, relative to the first power state. For example, if the transition is to occur after completion of the delivery of radiotherapy treatment to a patient, the transition may be from a relatively high power state to a relatively low power state.

The first, lower power state in the present example is a state in which the radiotherapy device 100 is maintained at a sufficiently high temperature, to enable it (on demand) to energise the magnets of the radiotherapy device 100 relatively quickly, and so to transition to an energised, beam-loaded state relatively quickly. However, when the device 100 is in the lower power state, the magnets are not yet sufficiently energised and so the radiotherapy beam is not yet loaded. The lower power state inherently consumes less energy per unit time than the energised, beam-loaded state does.

The higher power state in this example corresponds to the energised, beam-loaded state. When it is in the higher power state, the radiotherapy device 100 is in a standby mode, or preparatory mode, and so is able to "switch on" and deliver one or more radiotherapy beams quickly, for example within a few seconds. When it is delivering radiotherapy beams, the device 100 will be in a beam delivery power state, which consumes more energy per unit time than both the aforementioned lower power state and higher power state.

Optionally, at block 430 the computing system 210 is configured to communicate the control signal, to the radiotherapy device 100, in order to implement the transition from the first power state, to the second power state. In other words, optionally it can instruct the device 100 in this example to load the beam, in preparation for radiotherapy treatment. In other examples, this step 430 may comprise instructing the device 100 to take another action, such as (by way of non-limiting example) to switch off or power down the beam, for example once treatment is complete.

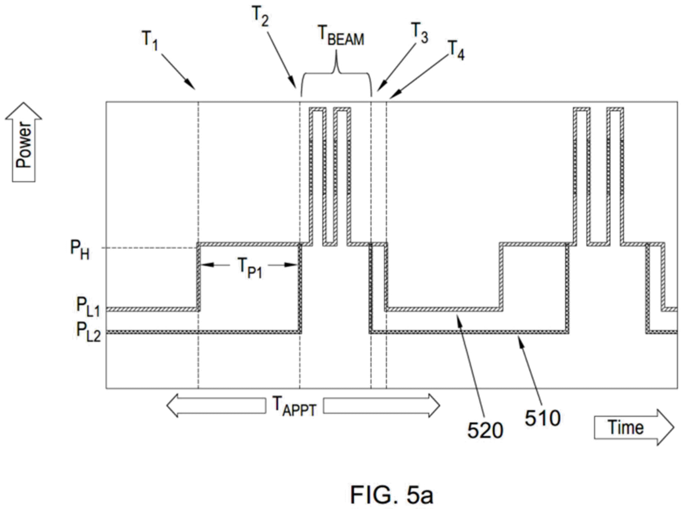

Optionally, at block 440, the computing system 210 is configured to cause the radiotherapy device 100 to deliver a beam or beams of radiotherapy treatment to the patient, in accordance with the control signal. The radiotherapy treatment may be defined by a treatment plan and/or by a proposed workflow for the device 100. The control signal may have been generated, and/or the timing of the delivery of the control signal to the radiotherapy device 100 may have been controlled, taking such a proposed workflow into account, as will be understood from the introductory portion of the present detailed description and further from the description of Figures 5a to 5c, herebelow.

Figure 5a is a first example of a power profile - i.e., of a change in power, over time - for the radiotherapy device 100, when the method 400 of Figure 4 is implemented for a patient during a treatment slot, or appointment. This is shown by way of example only and should not be regarded as limiting. In this example, the transition is from a first, lower power state to a second, higher power state. As detailed above, the present disclosure is applicable to transitions between other power states, including from relatively high to relative low power states, and vice versa.

The first power profile 510 that is achieved when the method 400 of Figure 4 is implemented is shown alongside a second, conventional power profile 520, which is to be expected when known, conventional approaches to control of a radiotherapy device, during a patient treatment slot or appointment, are implemented. Again, it will be appreciated that this is an illustrative example only. Specific details - such as power levels and the durations (periods of time) for which the device is at each respective power level - should not be regarded as limiting on the present disclosure.

As can be seen from Figure 5a, conventionally, a radiotherapy device is kept at a low power state P_{L1} - in this example, P_{L1} is a 4KW power state, but this number should not be regarded as limiting - between successive treatment slots. In other words, the device is powered down (but not switched off) between respective patient appointments. Conventionally, the radiotherapy device is then powered up to a higher power state P_{H} relatively quickly, at or towards the beginning of a patient treatment slot, or appointment. This higher power state P_{H} corresponds to the magnets of the device being energised and the beam being loaded, ready for delivery to the patient. For example, the higher power state may be between 6kW and 8kW, but this numerical range should not be regarded as limiting. The higher power state P_{H} may be regarded as being a standby, or preparatory, or prepared, state of the radiotherapy device.

It has been recognised herein that conventional approaches may lead to a radiotherapy device being powered up quite early, during a treatment slot - shown as time T1 in Figure 5a - and to there being a relatively long time period (T_{P1}), during which the device is in the higher power state P_{H} but beam delivery is not yet required. Therefore, the overall energy consumption of the device, across the treatment slot, may be quite high. According to the present disclosure, it has been recognised that it is possible to keep a radiotherapy device in a relatively low power state P_{L2}, which may be lower than the conventional low power state P_{L1} - in this example, P_{L2} is a 3KW power state, but this number should not be regarded as limiting - between treatment slots, and for more time, during a treatment slot, than has conventionally been done. It has further been recognised that a radiotherapy device can then be powered up to a higher power state P_{H} at a later time - shown as time T2 in Figure 5a - just before beam delivery is required.

It has been recognised herein that something other than the beginning of a treatment slot, or appointment, may be used to prompt, or trigger, the power changes of a radiotherapy device, or system, between a relatively low power state and a relatively high power state. This includes the recognition that there is typically some lag time between the beginning of a treatment slot and a time, or time period (T_{BEAM}), at/during which the radiotherapy treatment beam(s) will be applied. For example, there may be time required for information exchange between the patient and the device operator, before beam delivery. For example, there may be time required for patient positioning and/or image acquisition and/or image review steps, and/or adaptive imaging, before beam delivery. Therefore, it has been recognised herein that a 'trigger event', which may be a milestone or event within the treatment slot, may be used, to prompt beam loading and powering-up of the radiotherapy device, to reduce its overall energy consumption.

In the example of Figure 5a, a trigger event is defined as the closing of the door or doors of the patient treatment location. This seals off the patient treatment location, and so is an indication that it is now safe to deliver the radiotherapy treatment beams to the patient. It will be understood that, by the time (T2) the doors close, the patient will be *in situ* - for example, on the patient positioning surface 114 of the radiotherapy device 100. It will be understood that the operator of the radiotherapy device 100 should have left the patient treatment location, before the doors are closed. Therefore, the computing system 210 uses the doors closing at time T2 as a trigger event, to initiate the generation of a control signal that is configured for powering up the radiotherapy device, to transition it from a lower power state P_{L2} to a higher power state P_{H.} The control signal in this example is delivered to the radiotherapy device just after (or, substantially simultaneously with) the occurrence of the trigger event at time T2. However, in other examples there may be a (longer) delay between the trigger event and either the generation of the control signal and/or the application of the control signal, to the radiotherapy device 100.

According to the present disclosure, once the device is in the higher power state P_{H} in the example of Figure 5a, the device 100 is quickly controlled to deliver the radiotherapy beams to the patient, during a beam delivery time period T_{BEAM}. The first power profile 510 and the second, conventional power profile overlap with one another during the beam delivery time period T_{BEAM} in Figure 5a. Two distinct beam deliveries are shown within T_{BEAM} in Figure 5a, but it will be appreciated that this is illustrative only and should not be regarded as limiting. The device 100 will be in a beam-delivery power state during beam delivery. In this example, this may be around 12 kW, but this number should not be regarded as limiting on the present disclosure.

After beam delivery, the device 100 is powered down, to revert to the lower power state, at a time T3, when the delivery beam is terminated - i.e., when the beam treatment has completed. It can be seen from Figure 5a that, conventionally, a radiotherapy device may not be powered down until a later time T4, which is a time at which the beam is cleared - i.e., when the beam is reset so no energy is loaded. In some examples, when beam clear occurs, a set of default values are loaded and the radiotherapy system is ready to receive instruction regarding the next beam. But it has been recognised herein that there is no need to wait for T4, to power-down the device 100.

Although not shown in Figure 4, the method 400 therein may comprise generating a second control signal, configured for transitioning the device 100 back down to the lower power state P_{L2} from the higher power state P_{H} after beam delivery.

The method 400 may be repeated for an individual patient and/or it may be repeated for different respective patients. It will be appreciated that, for each patient, and potentially for each treatment slot, or appointment, which an individual patient attends, the details of the radiotherapy treatment provided may differ. In other words, each patient will usually have an individual treatment plan, for each of their radiotherapy treatment appointment(s).

A treatment plan for a patient - and/or the steps that should be carried out, during a patient treatment slot, or appointment - may be reflected in a workflow, for the radiotherapy device, for that respective patient treatment slot, or appointment. The workflow may be referred to as a proposed workflow. It is possible that changes may be made to a proposed workflow in real time (or, during the course of a treatment slot or appointment), for example due to medical reasons for the patient and/or for operational reasons for the device and/or for scheduling reasons and/or at the discretion of the operator of the radiotherapy device and/or due to another input.

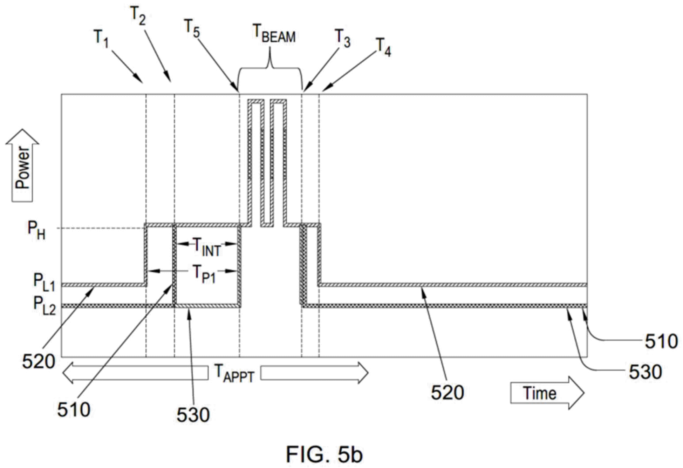

It has been recognised herein that a workflow, or proposed workflow, may be used to better control the configuration of - i.e., the generation and/or the timing of, and/or the communication, to the radiotherapy device, of - a control signal for transitioning the radiotherapy device from a first power state to a second, different power state, for example in readiness for radiotherapy beam delivery or to power-down after radiotherapy beam delivery. The computing system 210 (or other suitable controller or processor) that implements the method disclosed herein may have knowledge of the whole workflow, or of at least part of it, or of an indicator of at least part of the workflow, to enable it to better configure the control signal, for transitioning the device between its possible respective power states. An example of this is shown in Figure 5b.

As shown in Figure 5b, a trigger event, which in this non-limiting example comprises the doors closing to the patient treatment location, may occur at a time T2. In this example, however, the control signal is not yet generated - or, is at least not yet communicated to the radiotherapy device - at time T2. Instead, the control signal is generated and communicated to the radiotherapy device, to prompt the transition from the lower power state to the higher power state, at a later time, T5. This is because a workflow for the device in this example includes an interim step - after door closing and before beam delivery - of imaging, and possibly also of patient position correction (PPC). Imaging and PPC is an example interim step only - other patient workflows may have any combination of this and/or other steps. The method disclosed herein uses the knowledge of this interim step - which may include an expected, or demanded, or sensed, or actual time frame (T_{INT}), for carrying out the interim step - to configure the control signal accordingly, and so not to power-up the device too early. Instead, the powering-up of the device happens during a latter part of, or (as shown in Figure 5b) immediately after the completion of, the interim step - this is illustrated by a third power profile 530 in Figure 5b. This later powering-up of the device as shown in the third power profile 530 avoids the beam being loaded when it is not yet needed, during the interim step, and so reduces overall energy consumption for the device 100. Once the interim step has been completed and the device 100 powered up, the beams can be delivered, and the device subsequently powered down, in a similar manner as described above in relation to Figure 5a. The first power profile 510, the third power profile 530, and the second, conventional power profile overlap with one another during the beam delivery time period T_{BEAM} in Figure 5b.

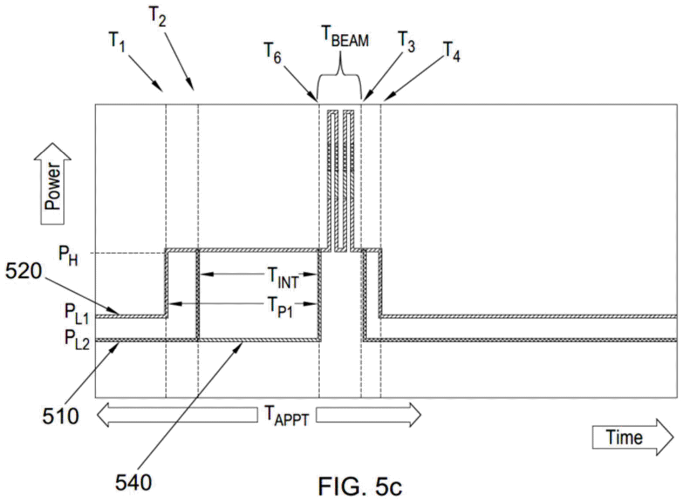

Figure 5c shows another example, comprising a fourth power profile 540, in which a proposed workflow, in combination with a trigger event, is used to configure a control signal for the radiotherapy device 100.

As with Figure 5b, in the example of Figure 5c, there is at least one interim step, between the trigger event of the doors closing at time T2 and the beam delivery at time T_{BEAM}. In this example, there are two interim steps - the first is an imaging and patient positioning correction (PPC) step, and the second is a plan review and adaptation step. These are example interim steps only - other patient workflows may have any combination of these and/or other steps. The method disclosed herein is implemented so that the powering-up of the device happens during a latter part of, or (as shown in Figure 5c) immediately after the completion of, the interim steps - at time T6. Time T6 is later (within its respective appointment slot) than time T5 is (within its respective appointment slot), because more interim steps are done, in Figure 5c as compared to Figure 5b, before the respective device is powered up. Again, this avoids the beam being loaded when it is not yet needed, during the interim steps, and so reduces overall energy consumption for the device 100. Once the interim steps have been completed and the device 100 powered up, the beams can be delivered, and the device subsequently powered down, in a similar manner as described above in relation to Figures 5a and 5b. The first power profile 510, the fourth power profile 540, and the second, conventional power profile 520 overlap with one another during the beam delivery time period T_{BEAM} in Figure 5c.

In the example of Figure 5c, the plan review and adaptation interim step may give rise to a change being suggested, or required, to the proposed workflow. This change may be input by a user or may be generated by the computing system 210, or by another computer or processor or controller, which communicates with the computing system 210. The control signal may be configured to take account of such a change. For example, the timing of the powering-up of the device 100, and/or the power level to which it is powered-up, and/or the duration of time for which it is powered-up, may be amended. A change to the proposed workflow may result in an existing control signal being amended, before it is communicated to the radiotherapy device. A change to the proposed workflow may result in a new control signal being generated, or configured. A change to the proposed workflow may prompt a return to one or more previous workflow steps. A change to the proposed workflow may comprise interrupting or changing a workflow when it has already begun.

In some cases, the doors closing will not be regarded as a trigger event - or, at least will not be regarded as the sole trigger event - to prompt generation and/or communication of the control signal disclosed herein. In such cases, another event, external or internal to the radiotherapy device, may be regarded as the trigger event. In some cases, a step or part of the proposed workflow may be regarded as the trigger event. In some cases, a user input will be required, for example to confirm or validate or complete a trigger event.

As described above in relation to Figure 2, the computing system 210 may be communicatively coupled to an input device 220 and/or an output device 230, via any suitable input/output circuitry 217. In some implementations, the input device 220 and/or the output device 230 may be elements of the computing system 210. A user - such as the operator of the radiotherapy device - may, in some cases, provide one or more inputs, which may influence the configuration of the control signal. For example, a user input may prompt a change to a timing of the radiotherapy delivery and/or to the power state to which the device is transitioned, and/or to any other suitable factor. The user may provide an input to the computing system 210, to provide scheduling information or other information, of which the system is not yet aware. The user may be able to override a planned operation of the computing system 210, for example for safety reasons and/or if changes are made the that computing system 210 has not yet allowed for.

User feedback may be provided, regarding the operation of the radiotherapy device or system. That user feedback may prompt a user to make a change to a workflow and/or to the current operation state of the radiotherapy device. An output may, in some cases, prompt the user to make a change to the radiotherapy device. For example, if an output indicates that the device is currently in a relatively high power state, and the user determines that this is unnecessary and/or unsafe, the user may be able to provide an input to lower the power state of the device.

A user may be able to work in harmony with the computing device 210, to choreograph the control and/or the operation of the radiotherapy device 100. A user may be able to control the radiotherapy device 100 to be in a state of their choosing - for example if there is a change in the day's schedule and/or an event within the day, or within a treatment session, of which the computing device 210 or other controller is not yet aware. For example, there may be a break between treatment sessions, for which the user can power-down the radiotherapy device to a suitable level.

The radiotherapy system may be configured to require an initial 'start of day' power state. For example, part or all of the radiotherapy system may be required to 'warm up' to a certain temperature or power state or other operational state, before it can be powered down to a lower power state, in preparation for a subsequent beam delivery.

Thus, method and systems are disclosed herein, which enable energy-efficient, sustainable, repeatable, and timely delivery of a radiotherapy beam or beams, to a patient. A trigger event may be defined and may be used, to prompt generation of a control signal for transitioning a radiotherapy system from a power save state to a standby, or prepared, state, from which beam delivery is possible, or vice versa. An indicator of a proposed workflow may be used, to help inform and guide the configuration of the control signal. The radiotherapy device may be operated, and/or radiotherapy may be delivered to the patient, based at least in part on the generated control signal.

### A power consumption metric for a medical device

Above, the disclosure has focused primarily on a first aspect, that of generating a control signal for a radiotherapy system. According to a second aspect, this disclosure also relates to a computer-implemented method for determining a power consumption metric for a medical device, and in particular for a radiotherapy device.

Promoting sustainable energy consumption behaviour in healthcare is becoming increasingly important. As environmental and so-called 'green' or 'eco' targets become more important to governments, health authorities and individuals, it becomes more important to analyse, monitor and improve the eco/green aspects of medical devices. In addition, across the globe, electricity costs are rising. Reducing power consumption is becoming essential for hospitals and businesses to not only reduce their carbon footprints, but also to save costs.

Numerous technologies have been developed to help hospitals save energy. However, an issue is that energy use of various medical devices is often "hidden" or not immediately obvious to most hospital staff. Additionally, there is typically a lack of information about the energy costs associated with specific activities, making it nearly impossible for most users of healthcare equipment to make choices which can help to reduce power consumption.

One example area in which reduced power consumption would be beneficial is the field of radiotherapy devices. Radiotherapy can be described as the use of ionising radiation, such as X-rays, to treat a human or animal body. Radiotherapy is commonly used to treat cancer, for example to treat tumours within the body of a patient or subject. In such treatments, ionising radiation is used to irradiate, and thus destroy or damage, cells which form part of the tumour.

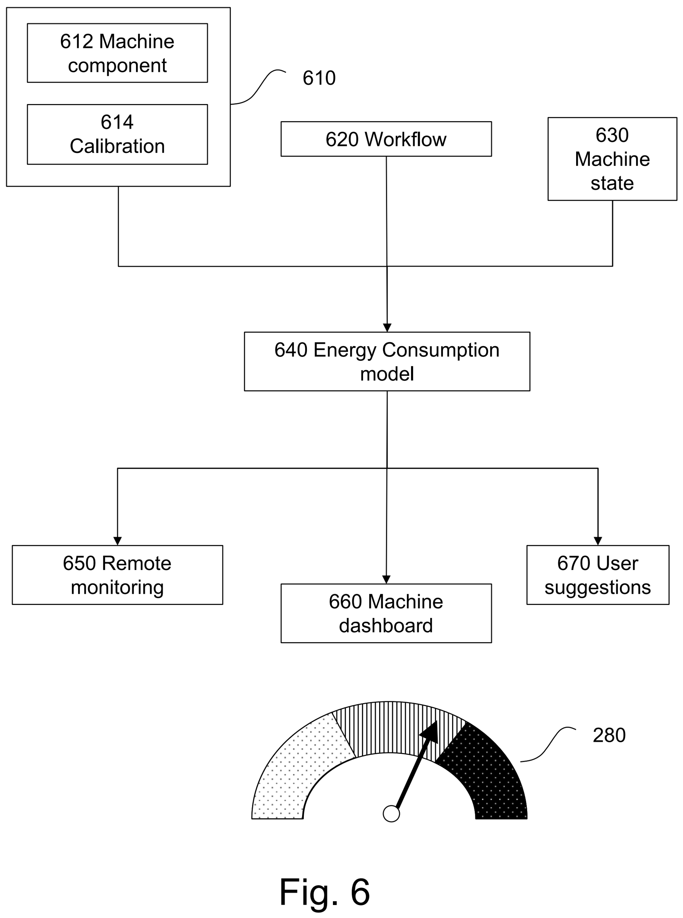

Figure 6 depicts possible inputs and outputs for an energy consumption model 640 suitable for use with a radiotherapy device.

One of the inputs to the energy consumption model 610 is machine state data 630. The machine state data 630 indicates which state of a plurality of different possible operational states the machine is currently operating in. Each of these states is associated with different power-consuming components. In other words, during each of these machine states, different components which make up the radiotherapy device are consuming power. Therefore, each machine state is associated with a different power usage level.

Example machine states which the radiotherapy device may be operating in are shown in table 1 below.

**Table 1: Possible operational states for a radiotherapy device.**

| | |
|---|---|
| Warm-up | The beam delivery components are powered on and undergo 'warm-up' procedures. For example, the electron gun requires a warm-up period to achieve thermal stability, the magnetron needs time to reach the correct operating temperature, etc. |
| Imaging mode (Pre-treatment) | The imaging system of the device is powered on and is obtaining images. Because the imaging is taking place pre-treatment, the beam-delivery components are on 'stand-by', i.e. remain warmed up. |
| Stand-by | In this mode, the device remains 'warmed-up, with the magnetron and other components being maintained at a temperature to enable movement to the "Ready to beam-on" state, |
| Ready to beam-on | In this mode the accelerating, steering and focusing magnets are energised ready to accelerate and guide electrons in the waveguide. |
| Beam-on | During 'beam-on', the device is actively delivering radiation to a patient. This is the primary operational state during patient treatment. |
| QA mode | The device is in a state used for quality assurance tests to ensure it meets all operational and safety standards. |
| Sleep or 'low-power mode' | Most of the device components, systems and sub-systems are powered down, but a number of core components remain on in order to reduce the amount of time taken to return to device to an operational state such as "imaging mode" or "beam-on". |
| Off | All components, systems and sub-systems are powered down. |

One of the inputs to the energy consumption model 610 is machine configuration data 610. The machine configuration data 610 comprises information about the configuration of the radiotherapy device. The configurations of radiotherapy devices can vary significantly depending on the manufacturer, design, intended use, generation, etc. The machine configuration data 610 may comprise both machine component data 612 and calibration data 614.

The machine component data 612 comprises a listing of all components, including systems and sub-systems, that consume power during one or more machine states. For example, the machine component data 612 may indicate that the device comprises a particular imaging system (e.g. CT, CBCT, MR. etc.), a particular beam focusing system, a particular magnet acceleration system in the waveguide, a particular cyclotron type, a particular type of collimator such as a multi-leaf collimator, MLC, and so on. The components of each of these systems uses energy during operation of the radiotherapy machine.

The machine component data 612 of the radiotherapy device can vary between repair cycles, based on the way in which the device has been repaired and which components have been replaced, and also based on a generation of the device. For example, the machine component data 612 may specify which of several variants of possible machine configuration the present radiotherapy device is. For example, on a newer generation of radiotherapy devices, a particular machine component or sub-system may have been updated to a lower power consumption equivalent component or sub-system.

In summary, the machine component data 612 comprises a list, database or registry of machine components, where each of the components uses power during at least one mode of operation of the device.

The components listed in the machine component data 612 may also be organised into subsets, according to which components consume power during each operational state. In a simple example, the components which form part of the imaging system, and which form part of the rotatable gantry, will consume power while a CT image of the patient is being obtained. These components therefore form a subset which is associated with an imaging mode. The beam generation components may consume power at a "stand-by" level during the imaging mode, too, and therefore may also form part of the same subset.

The calibration data 614 comprises at least one power usage value for each component. The power usage values are indicative of the amount of power required to operate the associated component, i.e. the power usage values are indicative of the amount of power used by the component during operation. For example, the calibration data 614 may comprise values in Amps or Watts.

Example calibration data is shown below in table 2.

**Table 2: example calibration data for a radiotherapy device.**

| **Component / Sub-System** | **Power Usage During Operation (W)** |
|---|---|
| Linear Accelerator (Linac) | 10,000 (at high energy beam) |
| | 9,000 (at low energy beam) |
| | 2,000 (stand-by) |
| Beam Modulator | 1,500 |
| Imaging System (e.g., CT Scanner) | 5,000 |
| Patient Positioning System | 500 |
| Cooling System | 2,000 |
| Control System | 700 |
| Treatment Table | 650 |
| Safety and Monitoring Systems | 200 |
| Power Supply Unit | 400 |
| Dosimetry Equipment | 150 |
| Steering Magnets | 1,000 |
| Magnetron | 3,000 (during 'beam-on' high energy) |
| | 3,000 (during 'beam-on' low energy) |
| | 700 (while warmed-up but not emitting RF, e.g. during 'stand-by' mode) |

As can be seen from inspection of the example entries for the linac and the magnetron, the calibration data 614 may also list multiple power usage values for each component, depending on the machine state. In this example, the beam generation system is capable of delivering a beam at two different energies ('high' and 'low'), and the beam generation components use different amounts of power during beam-on in both of these machine states. The magnetron consumes different amount of power during beam-on, and during stand-by mode.

In other words, the calibration data may comprises, for a first component of the plurality of components, both a first and a second power usage value, wherein the first power usage value is associated with a first operational state and the second power usage value is associated with a second operational state. The first component uses power in accordance with the first power usage value while the machine is in the first operational state, and the second component uses power in accordance with the second power usage value while the machine is in the second.

Optionally, one of the inputs to the energy consumption model 640 may be workflow data 620. The workflow data 620 comprises a schedule of upcoming treatment actions, such as "patient set-up", "patient imaging", "delivery of radiation according to treatment plan", etc. Each treatment action is associated with one or more recommended operational states. The workflow data 620 may be described, therefore, as a schedule of upcoming operational states.

For example, at the start of the day, the workflow data might indicate that the radiotherapy device is to undergo a daily QA check at a first scheduled time in the morning, during which time the device is powered on and goes through initial checks and warm-up procedures. At a second scheduled time, a first patient is scheduled for set-up, during which time the radiotherapy device is recommended to be in "imaging mode". A next treatment action might be delivering radiation to the first patient according to their treatment plan. During this treatment action, the radiotherapy device may move between "ready to beam-on (stand-by)" and "beam-on". The "beam-on" may be at a high energy or a low energy operational state, if the device is capable of different energy beams. The energy of beam used in the treatment will depend on the patient's treatment plan.

An example extract of suitable workflow data is shown below.

**Table 3: Example workflow for a morning of radiotherapy treatments**

| **Time** | **Treatment action / activity** | **Primary associated / recommended operational State(s)** |
|---|---|---|
| 8:00 - 8:15 AM | Machine QA, Warm-Up and Calibration | Warm-up |
| 8:15 - 8:30 AM | Patient 1 Setup and Imaging | Imaging mode |
| 8:30 - 8:45 AM | Treatment Delivery for Patient 1 | Ready to beam on |
| | | Beam on - energy 1 |
| 8:45 - 9:00 AM | Patient 1 Post-Treatment and Room Prep | Stand-by |
| 9:00 - 9:15 AM | Patient 2 Setup and Imaging | Imaging mode |
| 9:15 - 9:30 AM | Treatment Delivery for Patient 2 | Ready to beam on |
| | | Beam on - energy 1 |
| 9:30 - 9:45 AM | Patient 2 Post-Treatment and Room Prep | Stand-by |
| 9:45 - 10:00 AM | Patient 3 Setup and Imaging | Imaging mode |
| 10:00 - 10:15 AM | Treatment Delivery for Patient 3 | Ready to beam on |
| | | Beam on - energy 1 |
| 10:15 - 10:30 AM | Patient 3 Post-Treatment and Room Prep | Stand-by |
| 10:30 - 10:45 AM | Machine and System Check | QA mode |
| 10:45 - 11:00 AM | Patient 4 Setup and Imaging | Imaging mode |
| 11:00 - 11:15 AM | Treatment Delivery for Patient 4 | Ready to beam on |
| | | Beam on - energy 2 |
| 11:15 - 11:30 AM | Patient 4 Post-Treatment and Room Prep | Stand-by |
| 11:30 - 11:45 AM | Patient 5 Setup and Imaging | Imaging mode |
| 11:45 - 12:00 PM | Treatment Delivery for Patient 5 | Ready to beam on |
| | | Beam on - energy 1 |
| 12:00 - 12:15 PM | Patient 5 Post-Treatment and Room Prep | Stand-by |
| 12:15 - 1:00 PM | Break in treatment (e.g. for Lunch) | Sleep (low-power mode) |

The energy consumption model 640 may take the form of an algorithm, a trained Al/ML model, or the like.

In an example, the model can be used to generate, based on received machine state data and retrieved machine configuration data, a power consumption metric for the radiotherapy device which is indicative of the amount of power currently being consumed by the radiotherapy device.

If necessary, it can be determined from the machine state data which operational state the device is currently operating in. As noted above, each operational state of the plurality of possible operational states is associated with a respective subset of the plurality of components. The method may comprise retrieving component data which identifies the relevant subset of components which is associated with the operational state the machine is currently operating in. For example, for an operational state of "beam-on", the component data will identify components forming part of the beam generation system. The method also comprises retrieving calibration data comprising at least one power usage value for each of the components listed in the relevant subset of components, e.g. either for the components forming part of the beam generation system.

The power usage values are indicative of the amount of power required to operate the associated component, and therefore in a simple example, the energy consumption model algorithm may comprise summing each of the retrieved power usage values associated with the subset of components to provide a value indicative of the power currently being consumed by the radiotherapy device. The power usage values, rather than being measured in watts, may be values in amps or other values that enable an estimate of power consumption to be generated.

The power consumption value may be displayed to a user via a user interface, for example as part of a machine dashboard 660. The value may be displayed by an output / interface device such as a screen. The value may also be displayed in a graphical form 680, such as using a colour scheme where green is low power and red is high power, and/or using an arrow showing the current power consumption level as part of a range. The range may move from a minimum (with zero power being consumed), up to a maximum (e.g. the power typically consumed during the highest energy beam-on operational state).

The power consumption model may also be capable of outputting user suggestions 670, for example suggestions regarding an optimal state for the device to operate in based on the present treatment action specified by the workflow data, or a suggestion for a user to modify the current operational state to a different operational state based on a next scheduled treatment action.

The method may comprise determining that a current operational state of the radiotherapy device (determined from the machine state data 630) is different to a recommended machine operational state associated with the current treatment action, which information can be derived from the workflow data 620. According to the presently disclosed method, the recommended operational state (as determined from the workflow data 620) may be compared with the current operational state (as determined from the machine state data 630). If the state which the device is currently operating in is a higher power operating state than the recommended operating state, then the model may generate a suggestion that the user modify the state so as to be in line with the recommended state.

For example, the workflow data 620 may indicate that the current treatment action is 'Room Prep'. In this scheduled time block, the treatment room is cleaned and prepared for the next patient to enter. According to the workflow data 620, the recommended machine operational state associated with this scheduled action is "standby", in which components are maintained at an optimal operating temperature, but the steering and focusing magnets of the waveguide are not energised. If the machine state data 630 suggests that the machine is instead currently in the "ready to beam on" state, then the model outputs a suggestion that the user adjust the beam state to be "standby" in accordance with the recommended operational state, thereby saving power. The method may additionally comprise displaying the suggestion to the user via the user interface, along with a selectable control item such as a button on the interface which, when selected by the user, causes the radiotherapy device to enter the recommended operational state.

This kind of functionality enables users, e.g. physicians, to make intelligent choices about power usage.

The model may also make similar suggestions based on the next, or an upcoming, treatment action. For example, the suggestion may be for the user to modify the operational state, i.e. cause the device to enter a different operational state, to a recommended operational state associated with the next scheduled treatment action.

In an example, the next scheduled treatment action is a break in treatment (e.g. for lunch). This break in treatment does not require the radiotherapy device to be operational. This break in treatment is associated with a "low-power mode" machine state, where only a minimum number of components consume power. In this example, the suggestion is that the user should modify the current operational state to the "low-power mode" machine state, i.e. instruct or otherwise cause the device to enter the 'low-power mode" state. This acts as a reminder to the user and reduces the chances that they leave for lunch while keeping the device in an operational state that consumes more power than is required.

In some examples, the radiotherapy system may comprise one or more electronic visual displays, which enable clinicians to review treatment plans and/or to show data and graphs to the patient. In some treatment actions, these displays are not needed (e.g. if there is a break in treatment for lunch). This is an example of the type of component that might be turned off if a user follows the suggestion generated by the energy consumption model and causes the device to enter a lower-power consumption state.

The energy consumption model 640 may also provide data such as the current and past power consumption values to a remote server to enable remote monitoring 650 of the device performance. By collecting such data from each radiotherapy device in a fleet of such devices it is possible to identify outlying devices which are using more energy than other devices and/or which spend more time in higher-power consuming operational states.

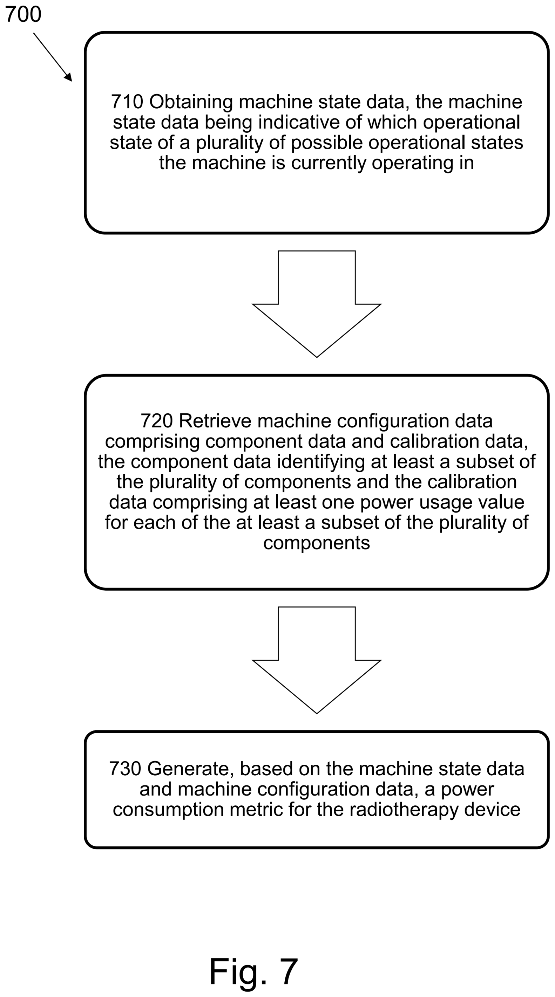

Figure 7 depicts a method 700 according to the present disclosure. The method is suitable for determining a power consumption metric for a radiotherapy device which comprises a plurality of components.

At block 710, machine state data is obtained. The machine state data is indicative of which operational state of a plurality of possible operational states the machine is currently operating in. The data might be received from a processor associated with the device, for example, which is configured to enable a user to instruct the device to enter particular operational states. This data may be received in real-time, or with a set frequency, so that the power consumption metric generated by the method 700 remains up to date.

At block 720, machine configuration data is retrieved which comprises component data and calibration data. The component data identifies at least a subset of the plurality of components, and the calibration data comprises at least one power usage value for each component in the at least a subset of the plurality of components.

At block 730, based on the machine state data and machine configuration data, a power consumption metric is generated for the radiotherapy device.

In a simple example of method 700, the machine state data indicates that the machine is currently operating in an 'imaging (pre-treatment)' mode. A subset of the component data which is associated with this state identifies that the radiotherapy device has a CBCT scanner with a certain number of components, and also indicates a number of beam generation components which consume power while the device is in this state. The calibration data lists a power usage value for each of these components and any other components which the component data indicates is associated with the 'imaging (pre-treatment)' state. Based on this information, a power consumption metric is generated. In particular, the power usage values associated with each of the components listed or otherwise identified in the component data are summed to provide a total power consumption value, which is an estimate of the total amount of power currently being consumed by the radiotherapy device.

By generating the metric based on machine state data and configuration data in the manner described herein, it is not necessary to monitor the actual power intake of the machine and its various systems and sub-systems. In this way, the need to use expensive and/or complex power monitoring equipment is avoided. In addition, the data and the model / algorithm is easily updateable upon service actions; for example if one component is replaced with another then it is straightforward to update the configuration data as needed.

By generating, and optionally displaying the metric, or otherwise making the user aware of the metric, the method helps to change user behaviour. As discussed above, the method may comprise generating a suggestion relating to power usage, in which case the method is useful in encouraging the user to push the machine into power saving mode if it is apparent that the suggested mode is beneficial to the environment and for electricity bills.

The algorithm / model and the machine data can be easily produced and updated during design and could be easily validated by testing against a traditional power monitoring solution.

A dashboard may be made available to the user that draws their attention to the power usage and actively draws their attention to "smart" power saving methods that the machine is configured to deploy to save power. Power-saving features of the device can be emphasised, in addition to the change in power consumption over machine generations.

Unless specifically stated otherwise, as apparent from the preceding discussion, it is appreciated that throughout the description, discussions utilizing terms such as " receiving", "determining", "comparing", "enabling", "maintaining," "identifying," "generating", "controlling", or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. A computer-implemented method of generating a first control signal for a radiotherapy system, the method comprising:
receiving an indicator of the occurrence of a trigger event; and,
in response to receiving said indicator of the occurrence of the trigger event, generating the first control signal, wherein the first control signal is configured to control a transition of the radiotherapy system from a first power state to a second, different power state.

2. The method of claim 1, wherein the first power state is a relatively low power state and the second power state is a relatively high power state, or vice versa.

3. The method of claim 1 or claim 2, wherein a proposed workflow is defined for the radiotherapy system, wherein the method comprises obtaining an indicator of the proposed workflow, and configuring the first control signal in accordance with said proposed workflow.

4. The method of any preceding claim, wherein the first control signal is configured to control a timing of the transition of the radiotherapy system, from the first power state to the second, different power state.

5. The method of claim 3, or of claim 4 when dependent on claim 3, wherein the proposed workflow comprises a radiotherapy beam delivery step for the radiotherapy system; and
wherein the first control signal is configured to cause said transition of the radiotherapy system, from the first power state to the second, different power state, to occur before a time at which the radiotherapy beam delivery step should occur, in accordance with the proposed workflow; or;
wherein the first control signal is configured to cause said transition of the radiotherapy system, from the first power state to the second, different power state, to occur after a time, by which the radiotherapy beam delivery step should be complete, in accordance with the proposed workflow.

6. The method of claim 5, wherein the time at which the radiotherapy beam delivery step should occur, and/or the time by which the radiotherapy beam delivery step should be complete, is defined based on one or more of:
the indicator of the occurrence of the trigger event;
an indicator that the proposed workflow has begun;
an indicator that a preceding step in the proposed workflow has begun;
an indicator that a preceding step in the proposed workflow is complete;
an indicator that an input has been received, from an operator of the radiotherapy system.

7. The method of claim 3 wherein the proposed workflow includes an interim step, which is scheduled to occur after the trigger event and before a radiotherapy beam delivery step; wherein the first control signal is configured to control the transition of the radiotherapy system to occur, from the first power state to the second, different power state, during or after the interim step of the proposed workflow.

8. The method of claim 7, wherein the proposed workflow comprises a set-up step, which demands a physical presence of an operator of the radiotherapy system, at a patient treatment location, before the trigger event; and/or wherein the interim step comprises at least one of: patient imaging; image review; patient position correction; review of a proposed beam delivery plan; and adaptation of a proposed beam delivery plan.

9. The method of any preceding claim, comprising receiving an indicator of a change to a proposed workflow for the radiotherapy system and generating the first control signal in accordance with said indicator of a change to the proposed workflow; and/or amending a previously-generated first control signal, in accordance with said indicator of a change to the proposed workflow.

10. The method of any preceding claim, comprising generating a second control signal, wherein the second control signal is configured to control a transition of the radiotherapy system from the second, different power state to the first power state.

11. The method of any preceding claim, comprising receiving an input from an operator of the radiotherapy system, and generating a third control signal, wherein the third control signal is configured to control a transition of the radiotherapy system from the first power state to the second, different power state, or vice versa, in accordance with said input.

12. The method of any preceding claim, comprising communicating the first control signal, to implement said transition of the radiotherapy system from the first power state to the second, different power state.

13. A control system for a radiotherapy system, the control system being configured to:
receive an indicator of the occurrence of a trigger event; and,
in response to receiving said indicator of the occurrence of the trigger event, generate a first control signal, wherein the first control signal is configured to control a transition of the radiotherapy system from a first power state to a second, different power state.

14. The control system of claim 13, wherein the control system is configured to communicate the first control signal, to implement said transition of the radiotherapy system from the first power state to the second, different power state, and optionally wherein the control system is configured to cause a radiotherapy beam to be delivered to a patient.

15. A computer readable medium storing instructions which when executed by one or more processors of a computing device, cause the computing device to carry out the method according to any one or more of claims 1 to 12.
